# EUROPEAN PATENT SPECIFICATION

(11) **EP 3 439 646 B1**
(45) Date of publication and mention of the grant of the patent: **16.06.2021**
(21) Application number: 16728411.6
(22) Date of filing: 04.04.2016
(51) Int. Cl.: A61K 31/14, A61K 31/202, A61K 31/7068, A61K 31/7072, A61P 25/00, A61K 31/714, A61K 33/18, A61K 33/30

(54) **METHOD FOR IMPROVING LANGUAGE SKILLS IN CHILDREN WITH SUSPECTED OR CONFIRMED CEREBRAL PALSY OR CHILDREN AT INCREASED RISK THEREOF**
VERFAHREN ZUR VERBESSERUNG DER SPRACHFÄHIGKEITEN BEI KINDERN MIT VERMUTETER ODER BESTÄTIGTER ZEREBRALER LÄHMUNG ODER KINDERN MIT ERHÖHTEM RISIKO DAFÜR
MÉTHODES POUR AMÉLIORER LES COMPÉTENCES LINGUISTIQUES CHEZ DES ENFANTS À INFIRMITÉ MOTRICE D'ORIGINE CÉRÉBRALE SUSPECTÉE OU CONFIRMÉE, OU CHEZ DES ENFANTS À RISQUE ACCRU DE CETTE DERNIÈRE

(43) Date of publication of application: 13.02.2019
(73) Proprietor: N.V. Nutricia, 2712 HM Zoetermeer (NL)
(72) Inventor: VOS, Arjen Paul, 3584 CT Utrecht (NL); COUNOTTE, Danielle Stefanie, 3584 CT Utrecht (NL); SMORENBURG, Ana Renée Pascale, 3584 CT Utrecht (NL)
(74) Representative: Nederlandsch Octrooibureau
(86) International application number: PCT/NL2016/050231
(87) International publication number: WO 2017/176108

(56) References cited:
- WO-A1-2012/125020
- WO-A2-01/78530
- Anonymous: "Product Information: Similac NeoSure Similac NeoSure Infant Formula with Iron", , 19 July 2016 (2016-07-19), pages 1-6, XP055313841, Retrieved from the Internet: URL:http://static.abbottnutrition.com/cms- prod/abbottnutrition.com/img/Similac-NeoSu re.pdf [retrieved on 2016-10-25]

## Description

The invention is in the field of medical nutrition and more particularly relates to a combination and composition for use in the treatment of language skills in children at risk of cerebral palsy (CP), or children with suspected or confirmed CP.

### BACKGROUND DESCRIPTION

Brain injury in the perinatal period or in early life of a child has a range of consequences dependent upon the exact timing, location and extent of the brain insult. Brain white matter appears particularly vulnerable to injury in the preterm period, whereas grey matter injury is typically the consequence of acute injury sustained around term birth. It is now recognized, however, that white and grey matter injury occur concurrently in pre-term and term brain injury. CP is a common sequelae of brain injury in the prenatal, perinatal or postnatal period. Although the brain injury resulting in CP is non-progressive, its manifestations vary throughout development. The incidence of CP worldwide is between 2.0-2.5 per 1000 live births, and CP is more common in infants born preterm, and in growth restricted infants.

Studies of improved early nutrition in preterm infants has shown improved neurodevelopmental outcomes in intervention groups, but whether increased nutritional intake in the first years of life in children with cerebral palsy improves growth or neurodevelopmental outcome has not been adequately studied.

During the last decennium, uridine, choline and omega-3 fatty acids such as DHA have attracted attention as active components in treating cognitive dysfunction, often in context of age-associated neurodegenerative processes. These compounds have been found rate-limiting precursors for membrane phosphatide synthesis. WO 2012/125034 describes a composition comprising: i) one or more of uridine and cytidine, or salts, phosphates, acyl derivatives or esters thereof; ii) a lipid fraction comprising at least one of docosahexaenoic acid (22:6; DHA), eicosapentaenoic acid (20:5; EPA) and docosapentaenoic acid (22:5; DPA), or esters thereof, in which the lipid fraction comprises less than 2 weight% of linolenic acid (ALA), calculated on the weight of all fatty acids; iii) choline, or salts or esters thereof; for use in the prevention or treatment of neurotrauma, traumatic brain injury, cerebral palsy and spinal cord injury. Further, WO 01/78530 describes infant formulas containing LC-PUFA for providing enhanced neurological development in infants. WO 2012/125020 describes a composition comprising DHA and/or EPA, uridine and/or cytidine and choline for use in the treatment or prevention of neurotrauma and cerebral palsy among others.

There is a need to further investigate whether dietetic and nutritional intervention helps improving neurodevelopmental outcomes in children who are at significant risk of neurological impairments, such as children suffering from cerebral palsy or children at increased risk thereof.

### SUMMARY OF THE INVENTION

The inventors have observed that upon administering a combination or composition comprising (i) one or more of uridine and cytidine, or salts, phosphates, acyl derivatives or esters thereof, (ii) docosahexaenoic acid (22:6; DHA), and/or eicosapentaenoic acid (20:5; EPA), or esters thereof, (iii) choline, or salts or esters thereof, language skills of children improve, wherein said composition or combination comprises: (i) 10 - 200 mg uridine monophosphate (UMP) per 100 kcal or per 100 ml; and (ii) 250 - 9000 mg DHA + EPA per 100 kcal or per 100 ml; and (iii) 60 - 350 mg choline per 100 kcal or per 100 ml. The language skills of children up to 42 months are preferably determined using the Bayley III scales of infant and toddler development as developed in Bayley "Bayley scales of infant and toddler development, third edition" (2006). In a setting of infants or toddlers at increased risk of developing CP, or diagnosed with CP, it was found that the intervention was particularly successful in improving language skills and language development measured at the language composite scale score of the Bayley Scales of Infant Development III.

The invention thus pertains to a composition or combination of (i), (ii) and (iii) for therapeutic use in improving language development and reducing language skills impairment in children with suspected or confirmed cerebral palsy, or children at increased risk thereof, preferably measured at the language scale of the Bayley III test, wherein said composition comprises:
(i) 10 - 200 mg uridine monophosphate (UMP) per 100 kcal or per 100 ml; and
(ii) 250 - 9000 mg DHA + EPA per 100 kcal or per 100 ml; and
(iii) 60 - 350 mg choline per 100 kcal or per 100 ml. The invention particularly pertains to said composition or combination of (i), (ii) and (iii) for therapeutic use in improving language development and reducing language skills impairment in children with suspected or confirmed cerebral palsy, or children at increased risk thereof, preferably measured at the language scale of the Bayley III test. It is preferred that therapeutically effective amounts of (i), (ii) and (iii) are part of a composition to be administered to said children, preferably in the form of a supplement to the child's normal diet.

### LIST OF FIGURES

Figure 1 shows the cognitive development score assessed with the Bayley III test of children at risk of CP at baseline, 12 and 24 months of control (placebo) or supplement administration. The result is clinically significant in favor of the supplement group.
Figure 2 shows the language development score assessed with the Bayley III test of children at risk of CP at baseline, 12 and 24 months of control (placebo) or supplement administration. A clinically significant higher language development score is observed in children receiving the supplement.
Figure 3 shows the motor development score assessed with the Bayley III test of children at risk of CP at baseline, 12 and 24 months of control (placebo) or supplement administration.
Figure 4 shows the cognitive development score assessed with the Bayley III test of children suspected of having CP or diagnosed with CP at baseline, 12 and 24 months of control (placebo) or supplement administration. The result is clinically significant in favor of the supplement group.
Figure 5 shows the language development score assessed with the Bayley III test of children suspected of having CP or diagnosed with CP at baseline, 12 and 24 months of control (placebo) or supplement administration. A clinically relevant higher language development score is observed in children receiving the supplement.
Figure 6 shows the motor development score assessed with the Bayley III test of children suspected of having CP or diagnosed with CP at baseline, 12 and 24 months of control (placebo) or supplement administration.

### DETAILED DESCRIPTION OF THE INVENTION

Throughout this application, the following terminology and abbreviations may be used;
Bayley III Test refers to the Bayley Scales of Infant Development version 3 (BSID-III), which is a standard series of measurements used primarily to assess the *motor* (fine and gross), *language* (receptive and expressive), and *cognitive* development of infants and toddlers at ages 16 days till 42 months. The primary purpose of the Bayley-III is to identify suspected developmental delays in children through the use of norm-referenced scores. Development of older children (> 42 months of age) may be tested with other tests such as a subset of tests directed at language development in the Kauffman Assessment Battery for Children (KABC II), verbal comprehension score of the Wechsler Intelligence scale for children, 4th edition and the British Picture Vocabulary Scale. The Bayley III Test consists of a series of developmental play tasks and takes between 45 - 60 minutes to administer. Raw scaled scores ranging from 1 to 19 of successfully completed items are converted to percentile rank scores and to composite scores. These scores are used to determine the child's performance compared with norms taken from typically developing children of their age (in months). In the context of the Bayley III test, age of preterm infants is corrected for gestational age, to compare their development with other infants. In studies including term control groups as a reference population, mean composite BSID-III scores for cognitive development are around 105, for language development around 109, and for motor development around 107 (Bos, A., Developmental Medicine & Child Neurology 2013, 55: 973-979). The composite scores between 85 and 115 are usually accepted as normal development (Bayley N. Bayley Scales of Infant and Toddler Development. 3rd edn. San Antonio, TX: Harcourt Assessment Inc, 2006).

A child at risk of developing CP may be a child already considered as suffering from delayed neurodevelopment. A child facing the risk of delayed neurodevelopment or suffering from delayed neurodevelopment, particularly at risk of developing cerebral palsy, may for example be a preterm infant, for reasons already accounted for elsewhere herein, or an infant being small for gestational age. Children at risk of developing CP can thus particularly pertain to one of the following groups: (1) born preterm and small for gestational age; (2) born preterm with brain injury; (3) born term but small for gestational age; (4) born term with brain injury (5); born preterm and small for gestational age with brain injury; and (6) born term but small for gestational age with brain injury.

Cerebral palsy (CP) refers to a group of permanent disorders of the development of movement and posture, causing activity limitation, that are attributed to non-progressive disturbances that occurred in the developing fetal or infant brain. The motor disorders of Cerebral Palsy are often accompanied by disturbances of sensation, perception, cognition, communication and behaviour, by epilepsy and by secondary musculoskeletal problems. Consequently, children at risk of cerebral palsy are at risk of developmental problems.

In one embodiment, children at risk of CP may be identified as children having early life brain injury, or who are at risk thereof. Early life brain injury provides a risk of developing CP. Early life brain injury may be caused by one or more of the following: lissencephaly, polymicrogyria, pachygyria, schizencephaly, periventricular leukomalacia, intracranial haemorrhage, intraventricular haemorrhage, basal ganglia lesion(s), thalamus lesion(s), cortico, subcortical lesion(s), hypoxic-ischemic encephalopathy, (perinatal) stroke, microcephalia vera, hemimegalencephaly, cortical dysplasia, heterotopias, hydranencephaly.

As used herein, a "child" of the present invention refers to children in early childhood, and includes, but is not limited to, an individual or subject of any sex that is of an age between the time of delivery to approximately 12 years after delivery. This term includes infants (from 0 to 12 months) and toddlers (between the ages of 12 and 42 months of age). In one embodiment, the preferred targeted children are up to 6 years, preferably up to 5 years, preferably up to 4 years of age, more preferably up to 42 months of age.

Language development refers to the acquisition and utilization of words, especially vocabulary. Language development is considered a measure of neurological development. Language development can be assessed by several methods including the Bayley III scale of infant development.

Preterm infant means a subject born before 37 weeks of gestational age. Within the group of preterms, preterms can be further differentiated in children born very preterm (28 to 32 weeks of gestational age) and extreme preterms (born before 28 weeks of gestational age). The term "preterm infant" is used interchangeably with "premature infant". Premature infants are at greater risk for cerebral palsy, delays in development, hearing problems, and problems seeing. These risks are greater the earlier a baby is born

"Small for gestational age" means an infant born smaller in size than normal for the gestational age, most commonly defined as a weight below the 10th percentile for the gestational age.

Term infant refers to an infant born between 37 and 42 weeks gestational age.

In one aspect of the present invention, (i), (ii) and (iii) are part of a composition according to the invention which is used as a pharmaceutical product comprising one or more pharmaceutically acceptable carrier materials.

In a preferred aspect of the present invention, (i), (ii) and (iii) are part of a composition which is used as a nutritional product, for example as a nutritional supplement, e.g., as an additive to a normal diet, as a fortifier, to add to a normal diet; or as a complete nutrition. Advantageously such supplement could be added in addition to the normal nutritional needs for that particular age, and the dosage could be adjusted thereto. A representation of the preferred amounts of the components (i), (ii) and (iii) in terms of the child's body weight is in accordance therewith.

A first aspect of the invention provides a combination or a composition comprising (i) one or more of uridine and cytidine, or salts, phosphates, acyl derivatives or esters thereof, (ii) docosahexaenoic acid (22:6; DHA), and/or eicosapentaenoic acid (20:5; EPA), or esters thereof, (iii) choline, or salts or esters thereof, for use for therapeutically improving language development and reducing language skills impairment of children at risk of or diagnosed with neurological and developmental impairment by feeding said child with said combination or composition, wherein said composition or combination comprises: (i) 10 - 200 mg uridine monophosphate (UMP) per 100 kcal or per 100 ml; and (ii) 250 - 9000 mg DHA + EPA per 100 kcal or per 100 ml; and (iii) 60 - 350 mg choline per 100 kcal or per 100 ml.

Preferably, the composition or combination of the invention comprises optionally (iv) one or more of vitamin B12, iodine, zinc; preferably for use in the prevention or treatment of impaired language development in children at risk of developing CP or children suspected of or diagnosed with cerebral palsy.

In one embodiment, neurodevelopment, such as at an (corrected) age of 12 months or 24 months, is assessed based on a score from Bayley Scales of Infant and Toddler Development (Bayley III). For the Bayley III test, the age of preterm infants is corrected for term gestational age to allow for a true comparison of development. Thus, at e.g. 12 months (corrected) age, neurodevelopment of an infant may be assessed and any neurodevelopmental delay may be detected. In one embodiment, said Bayley III score is based on assessment(s) of one or more of cognitive, motor, and language function(s) of the infant. It should be understood that, in the context of the present invention, these infants or children are targeted which are at increased risk of or suffering from neurodevelopmental delay in language function. Neurodevelopment in terms of language function may also be assessed using other methods but Bayley III. Such methods are considered known to the skilled person.

In one embodiment, the targeted child has a neurodevelopmental delay in language function which corresponds to a Bayley III language composite score of less than 85. In such embodiment, the child thus risks neurodevelopmental delay in language function corresponding to a Bayley III language composite score of less than 85.

In one embodiment the child is 0 to 12 years of age, preferably 0 to 6 years, preferably 0 to 5 years, more preferably 0 to 42 months.

Throughout the specification and claims, the dosages of the active components are expressed in terms of body weight. These can for instance be calculated based on WHO-provided infant/children average growth charts. However, it is preferred to administer the composition or combination according to the invention with the amounts as expressed throughout the specification and claims, capped at a maximum of 30 kg body weight. As an example, a child weighing 36 kilos (the average weight of an 11-year old child) may receive the preferred dosage for a 30 kg body weight child.

In one embodiment, the invention involves use of the combination or composition according to the invention in a child at risk of neurological impairment, particularly children with suspected or confirmed cerebral palsy, for improving language development, or reducing language impairment or dysfunction preferably as measured / confirmed by a language Bayley III score increase, more preferably by an increase of the score to reach 85 or more.

According to another embodiment, the composition or combination according to the invention is administered, preferably as a supplement, to a child at risk of or diagnosed with impaired language development, preferably daily, possibly more than once a day; preferably for at least 3 months, preferably at least 6 months, at least 9 months, at least 12 months, at least 15 months, at least 18 months, at least 21 months and more preferably at least 24 months. In one embodiment, the composition or combination is a daily dose unit, suitable for administration once a day.

Preferably, the composition or combination according to the invention comprises UMP and CMP

Preferably, the composition or combination according to the invention further comprises arachidonic acid.

Preferably, the composition or combination according to the invention further comprises iodine. In an embodiment of the invention a daily dose of the composition, or combination according to the invention preferably comprises between 10 to 50 µg per kg body weight iodine, preferably 12 to 48 µg per kg body weight, preferably 14 to 48 µg per kg body weight, more preferably 14 to 20 µg per kg body weight, more preferably 15 to 20 µg per kg body weight of iodine. In an embodiment the total daily dose of iodine does not exceed 450 µg.

Preferably, the composition or combination according to the invention further comprises zinc. Preferably the daily dose of the composition or combination according to the invention preferably comprises between 0.5 to 2.5 mg per kg body weight zinc, preferably between 0.6 and 2.2 mg per kg body weight, more preferably 0.6 to 1.0 mg per kg body weight, and even more preferably 0.7 to 1.0 mg per kg body weight of zinc. In an embodiment the total daily dose of zinc does not exceed 18 mg.

Preferably, the composition or combination according to the invention further comprises vitamin B12 or cobalamin equivalents thereof. Preferably the daily dose of vitamin B12 in the composition or combination according to the invention preferably is in the range of 0.05 to 1.0 µg per kg body weight, preferably 0.075 to 0.75 µg per kg body weight, preferably 0.09 to 0.5 µg per kg body weight, more preferably 0.09 - 0.2 µg per kg body weight, even more preferably 0.1 - 0.2 µg per kg body weight of vitamin B12.

Preferably, the composition or combination according to the invention further comprises one or more of: arachidonic acid; iodine; zinc; and vitamin B12. More preferably, the composition according to the invention further comprises arachidonic acid; iodine; zinc; and vitamin B12. Functional equivalents are encompassed within these terms.

Preferably, the composition or combination is provided as a complete nutritional product (comprising carbohydrates, fats and proteins) or as a nutritional supplement. Preferably the composition is a complete nutritional source by providing balanced amounts of all recommended nutrients .

The composition of the invention is an enteral composition, intended for oral administration. It is preferably administered in liquid form. In a preferred embodiment, the composition is a powder to be reconstituted with water prior to use. In an embodiment the enteral composition of the invention is an infant formula. In a preferred embodiment, the enteral composition of the invention is an infant formula, for use as sole source of nutrition. In a preferred embodiment, the composition is a nutritional supplement for use to supplement other dietary intake.

In another preferred embodiment, the enteral composition is a nutritional supplement or fortifier. In yet another embodiment the enteral composition of the invention is a tube feed.

In an embodiment the composition is a powder or liquid composition containing between 50 and 200 kcal per 100 ml, preferably between 65 and 150 kcal per 100 ml, more preferably between 75 and 100 kcal per 100 ml.

Further details of a composition of the invention are provided here below.

### ω-3 LC-PUFAs

The LCPUFAs (DHA, EPA and/or DPA) are preferably provided as triglycerides, diglycerides, monoglycerides, free fatty acids or their salts or esters, phospholipids, lysophospholipids, glycerol ethers, lipoproteins, ceramides, glycolipids or combinations thereof. Preferably, the present composition or combination comprises at least DHA in triglyceride form. Suitable ω-3 LCPUFA and/or DHA sources include tuna oil, (other) fish oils, DHA-rich alkyl esters, algae oil, egg yolk, or phospholipids enriched with ω-3 LCPUFA e.g. phosphatidylserine-DHA. Preferably, a composition or combination according to the invention comprises fish oil providing the omega-3 LCPUFA(s). Another particularly suitable source for the omega-3 LCPUFA(s) is algae oil.

Preferably, the total daily dosage of DHA+EPA+DPA taken together is in the range of 35 - 75 mg/kg body weight, more preferably 40 - 72.5 mg/kg body weight, even more preferably 43.5 - 72.5 mg/kg body weight, most preferably 43.5 - 60 mg/kg body weight, particularly 43 - 49.5 mg/kg body weight, whether or not the three (DHA, EPA and/or DPA) are present in the combination or composition according to the invention altogether. Preferably, these amounts are based on the total sum of DHA and EPA if present. In a preferred embodiment the total daily dose of DHA+EPA+DPA provided by thecombination or composition is at most 1500 mg.

DHA is preferably administered in a daily amount of at least 32.5 - 65 mg/kg body weight, more preferably 35 - 62.0 mg/kg body weight, even more preferably 36-60 mg/kg body weight, most preferably 36-50 mg/kg body weight, particularly 36-41 mg/kg body weight. In a preferred embodiment the total daily dose of DHA provided by the combination or composition is at most 1200 mg.

In terms of the composition or combination, the proportion of ω-3 LCPUFA (more preferably DHA+EPA+DPA, most preferably DHA+EPA) in the composition is preferably 1 to 95 wt%, more preferably 1 to 50 wt%, more preferably 1 to 30 wt% of the total fat. The present composition or combination preferably comprises 0.5 to 95 wt% DHA based on total fat, preferably 0.5 to 75 wt% DHA based on total fat, more preferably 1 to 60 wt%, even more preferably 1 - 50 wt%, more preferably 1 - 40 wt% based on total fat of the composition or combination. The present composition or combination preferably comprises 0.1 to 95 wt% EPA based on total fat, preferably 0.5 to 75 wt% EPA, even more preferably 0.5 - 50 wt%, more preferably 0.5 - 25 wt%, most preferably 0.5 - 15 wt%, based on total fat of the composition or combination.

In a combination or composition of the invention, DHA andor EPA is/are provided in an amount as described in Table 12.

In the combination or composition of the invention, the ratio of the weight of DHA to EPA is preferably larger than 1, more preferably 2:1 to 10:1, more preferably 3:1 to 7.5:1. The ratios take into account and optimize the balance between DHA and precursors thereof to ensure optimal effectiveness while maintaining low-volume formulations.

The DHA/AA weight ratio in the present composition or combination is preferably of at least 2, more preferably at least 5, preferably at least 6. If AA is administered, it preferably amounts to a daily amount of 2 - 35 mg/kg body weight, preferably 3 - 33 mg/kg body weight, more preferably 4 - 32.5 mg/kg body weight, even more preferably 4 - 8 mg/kg body weight.

### Uridine, cytidine and/or equivalents thereof

The uridine source is UMP and is provided in an amount as described in Table 12.

The present composition or combination comprises UMP, as UMP is most efficiently being taken up by the body. Hence, inclusion of UMP in the present combination and composition enables a high effectivity or efficacy at the lowest dosage and/or the administration of a low volume to the subject. Preferably at least 50 weight% of the uridine in the present combination and composition is provided by UMP, more preferably at least 75 weight%, most preferably at least 95 weight%. Doses administered are given as UMP. The amount of uracil sources can be calculated taking the molar equivalent to the UMP amount (molecular weight 324 Dalton).

In one embodiment, the present combination or composition of the invention comprises the sum of uridine and cytidine, including equivalents, in amounts of daily dosage of 3.0 - 8.5 mg/kg body weight, preferably 3.4 - 8.0 mg/kg body weight, more preferably 3.6 - 7.5 mg/kg body weight, even more preferably 3.6 - 5 mg/kg body weight, most preferably 3.6 - 4.4 mg/kg body weight.

The present method preferably comprises the administration of uridine (the cumulative amount of uridine, deoxyuridine, uridine phosphates, nucleobase uracil and acylated uridine derivatives). The preferred daily dosage of uridine would amount to 1.0 - 4.0 mg per kg body weight, preferably 1.0 - 3.5 mg per kg body weight, more preferably 1.5 - 3.0 mg per kg body weight, more preferably 1.8 - 3.0 mg/kg body weight, even more preferably 1.8 - 2.0 mg/kg body weight. The terms product, composition and combination are used interchangeably. In a preferred embodiment the total daily dose of uridine (equivalents) provided by the combination or composition is at most 625 mg.

In an embodiment, the present combination and composition according to the invention comprise (therapeutically) effective amounts of cytidine and/or an equivalent thereof, including salts, phosphates, acyl derivatives and/or esters. Preferably, a cytidine phosphate is selected from the group consisting of cytidine monophosphate (CMP), cytidine diphosphate (CDP) and cytidine triphosphate (CTP). The preferred cumulative daily dosage of cytidine amounts to 1.0 - 6.0 mg per kg body weight, preferably 1.0 - 5.0 mg per kg body weight, more preferably 1.5 - 4.8 mg per kg body weight, more preferably 1.8 - 4.5 mg/kg body weight, even more preferably 1.8 - 2.2 mg/kg body weight. These amounts are in addition to the amounts of uridine here above.

### Choline

The combination and composition according to the present invention comprise therapeutically effective amounts of choline, a choline salt and/or choline ester. Herein, the term 'choline' shall be considered to encompass all these equivalents. Choline salts are preferred. The choline salt is preferably selected from choline chloride, choline bitartrate, or choline stearate. The choline ester is preferably selected from the group consisting of phosphatidylcholine and lyso-phosphatidylcholine. The present composition comprises the administration of a daily dosage of preferably 5 - 40 mg per kg body weight per day. The daily amount of choline would amount preferably to 7.5 to 35 mg, more preferably 8 to 31 mg, more preferably 9 - 31 mg per kg body weight, most preferably 9 to 14 mg choline per kg body weight. The above numbers are based on choline, the amounts of choline equivalents or sources can be calculated taking the molar equivalent to choline into account, based on the molar mass of 104 g/mol choline. In a preferred embodiment the total daily dose of choline (equivalents) provided by the combination or composition is at most 400 mg.

In a combination or composition of the invention, choline is provided in an amount as described in Table 12.

### B Vitamins

Preferably, the present combination or composition according to the invention comprises therapeutically effective amounts of vitamin B12 (cobalamins). Functional equivalents are encompassed within these terms.

Vitamin B12 is preferably present in an amount to provide a daily dosage in the range of 0.05 to 1.0 µg per kg body weight, preferably 0.075 to 0.75 µg per kg body weight , preferably 0.09 to 0.5 µg per kg body weight, more preferably 0.09 - 0.2 µg per kg body weight. The term "vitamin B12" incorporates all cobalbumin equivalents known in the art. In a preferred embodiment the total daily dose of vitamin B12 (equivalents) provided by the combination or composition is at most 5 µg.

Preferably, in a combination or composition of the invention, vitamin B12 is provided in an amount as described in Table 12.

Preferably, in a combination or composition of the invention, zinc is provided in an amount as described in Table 12.

Preferably, in a combination or composition of the invention, iodine is provided in an amount as described in Table 12.

The composition may further comprise a digestible carbohydrate fraction, preferably including a source of lactose and a source of polysaccharide.

The combination or composition of the invention comprises:
- 10 - 200 mg UMP per 100 kcal or per 100 ml, and
- 60 - 350 mg choline per 100 kcal or per 100 ml, and
- 250 - 9000 mg DHA + EPA per 100 kcal or per 100 ml, preferably comprising 250 - 900 mg DHA per 100 kcal or per 100 ml; and/or preferably comprising 50 -150 mg EPA per 100 kcal or per 100 ml.

Optionally the combination or composition of the invention further comprises:
- 1 - 5 µg vitamin B12 or equivalents thereof per 100 kcal or per 100 ml, and
- 60 - 150 µg iodine per 100 kcal or per 100 ml, and
- 5 - 25 mg zinc per 100 kcal or per 100 ml.

In one aspect of the invention, the compositions either as complete nutrition or as a supplement as detailed above are intended for use in the treatment or prevention of language skills impairment. The composition may in particular be used in children with CP and children suspected of or at risk of CP. The compositions may in particular be used in the treatment of language development impairment.

### EXAMPLES

For a more complete understanding of the present disclosure, reference is now made to the following examples taken in conjunction with the accompanying drawings.

### Example 1 - clinical trial study with children at risk of CP

In a two-year blind randomized placebo-controlled trial it was investigated whether postnatal dietetic and nutritional intervention, with neurotrophic supplementation, improves growth and neurodevelopmental outcomes in children who are at significant risk of neurological impairment and global developmental delay. Sixty children at risk of cerebral palsy were randomized to receive postnatal dietetic and nutritional intervention, with either a neurotrophic supplement or a placebo substance. Both groups received the feed supplement daily. The included children fall in 3 broad categories (i) very preterm (born before 31 weeks of gestation) and small for gestational age, (ii) preterm with brain injury, (iii) term with brain injury, and were included based on the following criteria:

### (i) Birth ≤30 weeks gestation:

- Small for Gestational Age - weight less than 9^{th} centile; OR
- Grade IIa, IIb, III or IV Germinal Matrix Haemmorhage (GMH, germinal matrix hemorrhage) - Intra Ventricular Haemorrhage (IVH) or pathological periventricular flare / leucomalacia;

### (ii) and (iii) Birth 31-42 weeks gestation:

- Hypoxic Ischaemic Encephalopathy Sarnat Grade II and III; OR
- Grade IIa, IIb, III or IV GMH-IVH or pathological periventricular flare / leucomalacia; OR Ultrasound scan flares;
- Magnetic resonance imaging (MRI) abnormalities: Posterior limb of the internal capsule (PLIC), basal ganglia, thalami, white matter and cortex.

### Exclusion criteria:

### Preterm birth with

- Grade 1 GMH - IVH or pathological periventricular flare / leucomalacia;

### General exclusion criteria

- Progressive neurological conditions
- Gastrointestinal disease which significantly impairs absorption
- Multiple congenital abnormalities or syndromic associations
- Such low hearing that assessment with the Bayley scales cannot be completed
- Parents considered by clinicians to be unable to follow study protocol

Neurodevelopmental development was assessed using the Bayley Scale of Infant Development (BSID III), at baseline, 12 and 24 months after enrollment in the study. This score is corrected for the age of the child in months at the time of the scoring.

Both groups received:
- Fortnightly dietetic review (in person or by telephone);
- Advice regarding feeding and optimizing both dietary macro (glucose polymers and/or long chain triglycerides) and micronutrient intake;
- The 'intervention' group was allocated to receive the neurotrophic feed supplement according to the invention (comprising choline, UMP, DHA, vitamins and minerals as further described in Table 1);
- The 'control' group was allocated to receive a placebo supplement (Table 1).

**Table 1A. Nutritional values of the neurotrophic feed supplement and control or placebo supplement and 1B. feeding schedule for children enrolled in the clinical trial receiving the study products.**

| *Nutrients* | **nutritional value active supplement** (501 kcal/100 g) | **nutritional value control supplement** (503 kcal/100 g) |
|---|---|---|
| **MACRO NUTRIENTS** | | |
| Energy | 501 kcal / 100 g | 503 kcal/100g |
| Protein | 2.2 g / 100 kcal | 2.2 g/100 kcal |
| Whey | 1.3 g / 100 kcal | 1.3 g/100 kcal |
| **Fat** | 5.0 g / 100 kcal | 5.0 g/100 kcal |
| Linoleic acid (LA) | 534 mg / 100 kcal | 693 mg/100 kcal |
| Alpha-linolenic acid | 77 mg / 100 kcal | 122 mg/100 kcal |
| Mysteric acid | 63 mg / 100 kcal | 29 mg / 100 kcal |
| Palmitoleic acid | 74 mg / 100 kcal | 8.2 mg / 100 kcal |
| Oleic acid | 1917 mg / 100 kcal | 2637 mg / 100 kcal |
| ALA | 77 mg / 100 kcal | 122 mg / 100 kcal |
| AA | 42 mg /100 kcal | 20 mg/100 kcal |
| EPA | 77 mg / 100 kcal | 0.8 mg/100 kcal |
| DHA | 377 mg /100 kcal | 3.7 mg/100 kcal |
| (n-6)/(n-3) ratio | 5.4 | 1.1 |
| LA/ALA ratio | 5.7 | 6.9 |
| **Carbohydrates** | 11.6 g /100 kcal | 11.7 g/100 kcal |
| Sugars | 8.9 g / 100 kcal | 9.1 g/100 kcal |
| Milk fat | 0.1 g / 100 kcal | 0.1 g / 100 kcal |

| **MINERALS** | | |
|---|---|---|
| Na | 25 mg / 100 kcal | 25 mg / 100 kcal |
| K | 93 mg / 100 kcal | 116mg / 100 kcal |
| Cl | 63 mg / 100 kcal | 63 mg / 100 kcal |
| Ca | 63 mg / 100 kcal | 63 mg / 100 kcal |
| P | 58 mg / 100 kcal | 40 mg / 100 kcal |
| Mg | 6.3 mg / 100 kcal | 6.3 mg / 100 kcal |

| **TRACE ELEMENTS** | | |
|---|---|---|
| Fe | 0.62 mg / 100 kcal | 0.63 mg / 100 kcal |
| Zn | 7.5 mg / 100 kcal | 0.63 mg / 100 kcal |
| Cu | 25 µg / 100 kcal | 25 µg / 100 kcal |
| Mn | 4.4 µg / 100 kcal | 4.4 µg / 100 kcal |
| Se | 1.3 µg / 100 kcal | 1.3 µg / 100 kcal |
| I | 150 µg / 100 kcal | 6.2 µg / 100 kcal |

| **VITAMINS** | | |
|---|---|---|
| Vitamin A | 75 µg RE/ 100 kcal | 75 µg RE/ 100 kcal |
| Vitamin D3 | 1.8 µg / 100 kcal | 1.8 µg / 100 kcal |
| Vitamin E | 1.4 mg α-TE / 100 kcal | 1.6 mg α-TE / 100 kcal |
| Vitamin K1 | 6.0 µg / 100 kcal | 6.7 µg / 100 kcal |
| Thiamin (B1) | 50 µg / 100 kcal | 50 µg / 100 kcal |
| Riboflavin (B2) | 110 µg / 100 kcal | 110 µg / 100 kcal |
| Niacin (B3) | 1.0 mg NE/ 100 kcal | 1.5 mg NE/ 100 kcal |
| Pantothenic acid (B5) | 375 µg / 100 kcal | 375 µg / 100 kcal |
| Vitamin B6 | 44 µg / 100 kcal | 44 µg / 100 kcal |
| Folic acid | 5.0 µg / 100 kcal | 5.0 µg / 100 kcal |
| Vitamin B12 | 1.2 µg / 100 kcal | 0.17 µg / 100 kcal |
| Biotin | 2.0 µg / 100 kcal | 2.0 µg / 100 kcal |
| Vitamin C | 10 mg / 100 kcal | 10 mg / 100 kcal |
| Choline | 105 mg / 100 kcal | 14 mg / 100 kcal |
| Taurine | 0.41 mg / 100 kcal | 0.41 mg / 100 kcal |
| L-Carnitine | 1.8 mg / 100 kcal | 1.8 mg / 100 kcal |
| Inositol | 7.2 mg / 100 kcal | 6.2 mg / 100 kcal |
| UMP | 18 mg / 100 kcal | 0 mg / 100 kcal |
| CMP | 18 mg / 100 kcal | 0 mg / 100 kcal |
| Total nucleotides | 36 mg / 100 kcal | 0 mg / 100 kcal |

**Table 1B:**

| **Body weight (kg)** | Required study product (gram / day) |
|---|---|
| < 1 | 2 |
| 1-1.25 | 2.5 |
| 1.25-1.5 | 3 |
| 1.5-1.75 | 3.5 |
| 1.75-2 | 4 |
| 2-2.25 | 4.5 |
| 2.25-2.5 | 5 |
| 2.5-3 | 6 |
| 3-3.5 | 8 |
| 3.5-4 | 9 |
| 4-5 | 11 |
| 5-6 | 13 |
| 6-7 | 14 |
| 7-8 | 16 |
| 8-9 | 18 |
| 9-10 | 20 |
| 10-11 | 22 |
| 11-12 | 24 |
| > 12 | 24 |

The results for the different development aspects tested with the Bayley III Scale are plotted in figures 1-3.

The median cognitive scores for the children at risk of neurological impairment enrolled in the study are shown in figure 1; table 2 shows the mean cognitive composite scores for these children adjusted for baseline measurement, treatment, visit, interaction between treatment and visit, and the minimisation factors neurological severity, gender and length of gestation at randomization. The scores are determined at baseline, 12 months and 24 months after enrollment in the study for both the group receiving placebo and the group receiving the supplement. No significant difference between the treatment groups at the different time points was detected, although a clinically significant trend towards a higher cognitive score at 24 months for the supplemented group is observed.

**Table 2: Bayley III cognitive score**

| *Visit* | *Numbers included** | | *Mean (SD)* * | | *Difference in means* |
|---|---|---|---|---|---|
| | *Placebo* | *Supplement* | *Placebo* | *Supplement* | *(95% CI)* * # |
| Baseline | 21 | 24 | 77.4 (14.2) | 75.8 (13.5) | |
| 12 month | 21 | 24 | 82.4 (20.1) | 85.2(18.1) | 4.4 (-4.7, 13.4) |
| 24 month | 19 | 24 | 81.6 (18.5) | 87.7(20.4) | 9.0 (-0.2, 18.2) |

| | | | | | |
|---|---|---|---|---|---|
| * All subjects included in analysis have a measurement for Bayley cognitive score at baseline and at least one other time point; # adjusted for baseline measurement, treatment, visit, interaction between treatment and visit, and the minimisation factors neurological severity, gender and length of gestation. | | | | | |

Bayley III cognitive scores have been re-assessed and corrected for the fact that one child had received supplement for two years despite being allocated to the placebo group.

**Table 2A therefore represents the statistics when using a per protocol analysis.**

| *Visit* | *Numbers included** | | *Mean (SD)* * | | *Difference in means* |
|---|---|---|---|---|---|
| | *Placebo* | *Supplement* | *Placebo* | *Supplement* | *(95% CI)* * # |
| Baseline | 20 | 25 | 77.0 (14.5) | 76.2 (13.3) | |
| 12 month | 20 | 25 | 82.0 (20.5) | 82.20 (17.8) | 5.4 (-3.7, 14.6) |
| 24 month | 18 | 25 | 80.8 (18.7) | 88.0 (20.1) | 10.6 (1.3, 19.9) |

The median performance of the same children on language development is shown in figure 2, mean language composite scores statistically adjusted for the same factors as the cognitive score are shown in table 3. A clinically significant improved language development score was observed over time in children that received the nutritional supplement as compared to the placebo group. A score above 85 is accepted as normal language development.

**Table 3: Bayley III language score**

| *Visit* | *Numbers included** | | *Mean (SD)** | | *Difference in* |
|---|---|---|---|---|---|
| | *Placebo* | *Supplement* | *Placebo* | *Supplement* | *means (95% CI)** # |
| Baseline | 21 | 24 | 67.5 (11.9) | 67.0 (9.6) | |
| 12 month | 21 | 24 | 74.6 (12.0) | 77.6 (14.6) | 2.7 (-6.7, 12.1) |
| 24 month | 19 | 24 | 83.2 (19.6) | 91.5 (20.1) | 8.6 (-1.1, 18.2) |

| | | | | | |
|---|---|---|---|---|---|
| * All subjects included in analysis have a measurement for Bayley language score at baseline and at least one other time point; # adjusted for baseline measurement, treatment, visit, interaction between treatment and visit, and the minimisation factors neurological severity, gender and length of gestation. | | | | | |

The performance of the same children on motor development scores is shown in figure 3, mean motor composite Bayley III scores adjusted for baseline measurement, treatment, visit, interaction between treatment and visit, and the minimisation factors neurological severity, gender and length of gestation are shown in table 4. No significant difference in motor development score was observed over time in children that received the nutritional supplement as compared to the placebo group. A score above 85 is accepted as normal motor development.

**Table 4: Bayley III motor score**

| *Visit* | *Numbers included** | | *Mean (SD)** | | *Difference in* |
|---|---|---|---|---|---|
| | *Placebo* | *Supplement* | *Placebo* | *Supplement* | *means (95% CI)** # |
| Baseline 12 | 21 | 24 | 82.2 (12.5) | 83.3 (8.8) | |
| month 24 | 20 | 24 | 80.0 (21.4) | 79.6 (18.3) | -0.2 (-10.6, 10.3) |
| month | 19 | 23 | 78.8 (21.6) | 77.8 (21.8) | -1.2 (-11.9, 9.5) |

| | | | | | |
|---|---|---|---|---|---|
| * All subjects included in analysis have a measurement for Bayley motor score at baseline and at least one other time point; # adjusted for baseline measurement, treatment, visit, interaction between treatment and visit, and the minimisation factors neurological severity, gender and length of gestation. | | | | | |

In summary, any effect of intervention on the performance of the same children in terms of cognitive score and language score is clinically significant over time in children that received the nutritional supplement as compared to the placebo group.

### Example 2 - clinical trial study with children suspected of or diagnosed with cerebral palsy

In a two-year blind randomized placebo-controlled trial it was investigated whether postnatal dietetic and nutritional intervention, with neurotrophic supplementation according to the invention (Table 1A and B), improves growth and neurodevelopmental outcomes in children who are between 1 and 18 months of age at inclusion in the study and have a suspected or confirmed clinical diagnosis of Cerebral Palsy according to the following definition: Children included suffer from a group of permanent disorders of the development of movement and posture, causing activity limitation, that are attributed to non-progressive disturbances that occurred in the developing fetal or infant brain. The motor disorders of cerebral palsy are often accompanied by disturbances of sensation, perception, cognition, communication, and behavior, by epilepsy, and by secondary musculoskeletal problems. Children with progressive neurological degenerative conditions, such low hearing they cannot complete assessment with the Bayley scales, children with gastrointestinal disease which significantly impairs absorption as well as children from parents that were considered unable to follow the study protocol were excluded from participation in the study.

Neurodevelopmental development was assessed using the Bayley Scale of Infant Development (BSID III), at baseline, 12 and 24 months after enrollment in the study. This score is corrected for the age of the child in months at the time of the scoring.

Both groups received:
- Fortnightly dietetic review (in person or by telephone)
- Advice regarding feeding and optimizing both dietary macro (glucose polymers and/or long chain triglycerides) and micronutrient intake.
- Both groups received a measured feed supplement (neurotrophic factors or placebo as described in Table 1A and B) to add to feed/food once a day

The treatment groups were balanced for the minimization factors: gender, cerebral palsy type, visual impairment and corrected age at time of recruitment. There were fewer subjects in the youngest (1-4 months) category for corrected age at time of recruitment than in the middle (6- 12 months) category in the placebo group whereas there were equal numbers of subjects who were 1-4 months and 6- 12 months at time of recruitment in the supplement group. The median gestational age at birth was 3 weeks lower in the supplement group.

The following table summarizes the characteristics of the groups assigned to either placebo or supplement at randomization.

**Table 5. Characteristics of children at baseline randomized to placebo or supplement**

| | Placebo | Supplement |
|---|---|---|
| | N or mean (SD) or median (LQ, UQ) | N or mean (SD) or median (LQ, UQ) |
| Numbers assigned treatment | 20 | 20 |
| Cerebral Palsy type: 4 limb involvement / other | 8/12 | 7/13 |
| Corrected age at time of recruitment: 1- 5 months / 6-12 months / 13-18 months | 2/8/10 | 5/5/10 |
| Sex: female / male | 6/14 | 6/14 |
| Gestational age at birth | 38.0 (32.5, 41.0) | 35.0 (28.0, 38.5) |
| Maternal educational qualification: GCSE / A level / College+ | 7/8/5 | 9/4/5* |
| Bayley cognitive score | 75.3 (17.5) | 72.5 (15.5) |
| Bayley language score | 74.7 (15.7) | 77.7 (13.9) |
| Bayley motor score | 63.4 (19.4) | 65.7(16.9) |

| | | |
|---|---|---|
| * Information missing for 2 subjects that received supplement | | |

The primary analysis was based on the intention-to-treat and involved all subjects who were randomly assigned and had information on Bayley cognitive score at baseline and at least one other time point (12 months or 24 months). Thus data from 32 subjects were available for the intention-to-treat analysis. Two subjects were considered protocol violators. One subject withdrew before supplementation but also has data at 12 months so was excluded from the per- protocol analysis. One subject was randomized to receive placebo but actually received supplement for the 2 years of follow-up so was analysed as having received supplement in the per-protocol analysis. Consequently 31 subjects remained in the per- protocol analysis that will be discussed below.

At baseline, 12 and 24 months all children participating in the follow-up underwent Bayley III assessment to assess their language, cognitive and motor skills.

The median Cognitive composite Bayley III scores for the children suspected of having CP or diagnosed with cerebral palsy enrolled in the study are shown in figure 4; table 6 shows the mean cognitive composite scores for these children. The scores are determined at baseline, 12 months and 24 months after enrollment in the study for both the group receiving placebo and the group receiving the supplement. An increase in Bayley III cognitive score was observed for children receiving the supplement as compared to children receiving the placebo.

**Table 6. Bayley III cognitive composite score**

| *Visit* | *Numbers included** | | *Mean (SD)** | | *Difference in means (95% CI)**# |
|---|---|---|---|---|---|
| | *Placebo* | *Supplement* | *Placebo* | *Supplement* | |
| Baseline | 18 | 14 | 73.6 (17.6) | 71.4 (14.7) | |
| 12 month | 18 | 14 | 71.1 (18.8) | 75.0 (15.8) | 4.6 (-2.4, 11.6) |
| 24 month | 16 | 13 | 72.2(19.8) | 77.7 (19.2) | 4.5 (-2.7, 11.8) |

| | | | | | |
|---|---|---|---|---|---|
| * All subjects included in analysis have a measurement for Bayley cognitive score at baseline and at least one other time point; # adjusted for baseline measurement, treatment, visit, interaction between treatment and visit, and the minimisation factors gender, cerebral palsy type, visual impairment and corrected age at time of recruitment. | | | | | |

The median performance of the same children on language development is shown in figure 5, mean language composite scores are shown in table 7. An improved language development score was observed over time in children that received the nutritional supplement as compared to the placebo group

**Table 7. Bayley III Language composite score**

| *Visit* | *Numbers included** | | *Mean (SD)** | | *Difference in means (95% CI)***#* |
|---|---|---|---|---|---|
| | *Placebo* | *Supplement* | *Placebo* | *Supplement* | |
| Baseline | 18 | 14 | 73.2 (15.9) | 77.1 (12.9) | |
| 12 month | 18 | 14 | 68.2 (21.0) | 74.8 (19.8) | 5.0 (-5.1, 15.1) |
| 24 month | 16 | 13 | 66.1 (24.9) | 78.5 (25.6) | 10.1 (-0.4,20.6) |

| | | | | | |
|---|---|---|---|---|---|
| * All subjects included in analysis have a measurement for Bayley language score at baseline and at least one other time point; # adjusted for baseline measurement, treatment, visit, interaction between treatment and visit, and the minimisation factors gender, cerebral palsy type, visual impairment and corrected age at time of recruitment. | | | | | |

The performance of the same children on motor development scores is shown in figure 6, mean motor composite Bayley III scores are shown in table 8. A clinically relevant difference (>10) in motor development score was observed at 24 months in children that received the nutritional supplement as compared to the placebo group. A score above 85 is accepted as normal motor development.

**Table 8. Bayley III Motor composite score**

| *Visit* | *Numbers included** | | *Mean (SD)** | | *Difference in* |
|---|---|---|---|---|---|
| | *Placebo* | *Supplement* | *Placebo* | *Supplement* | *means (95% CI)***#* |
| Baseline | 18 | 14 | 62.5 (20.0) | 62.3 (12.9) | |
| 12 month | 18 | 14 | 61.6 (17.0) | 67.4 (21.9) | 7.0 (-1.9, 15.9) |
| 24 month | 16 | 13 | 52.3 (18.9) | 63.1 (15.9) | 10.2 (0.9, 19.6) |

| | | | | | |
|---|---|---|---|---|---|
| * All subjects included in analysis have a measurement for Bayley motor score at baseline and at least one other time point; # adjusted for baseline measurement, treatment, visit, interaction between treatment and visit, and the minimisation factors gender, cerebral palsy type, visual impairment and corrected age at time of recruitment. | | | | | |

### Example 3 - Products for use in the treatment of cerebral palsy:

An exemplary product of the invention is a liquid formula comprising the active components according to table 9. It is a bottle of infant formula for use in children of 1 year and older, that provides additionally 450 mg DHA, 126 mg choline and 21.6 mg UMP

**Table 9.**

| *Nutrients* | **(200ml bottle); nutritional value** |
|---|---|
| **MACRO NUTRIENTS** | |
| Energy | 75 kcal/100 ml |
| Protein | 2.5-2.6 g/100 kcal |
| Whey | 0.5 (20%) g/100 kcal |
| Fat | 4.7 g/100 kcal |
| Linoleic acid | 575-1060 mg/100 kcal |
| Alpha-linolenic acid | 109-205 mg/100 kcal |
| AA | 34 mg /100 kcal |
| EPA | 60 mg/100 kcal |
| DHA | 300 mg/100 kcal |
| Carbohydrates | 11.9-12.0 g /100 kcal |
| Sugars | ≤5.0 g/ 100 kcal |
| MF | 1.0-2.0 g / 100 kcal |

| **MINERALS** | |
|---|---|
| Na | 44 mg / 100 kcal |
| K | 92 mg / 100 kcal |
| Cl | 66 mg / 100 kcal |
| Ca | 55-88 mg / 100 kcal |
| P | 50-67 mg / 100 kcal |
| Mg | 11.1-14 mg / 100 kcal |

| **TRACE ELEMENTS** | |
|---|---|
| Fe | 1.0 mg / 100 kcal |
| Zn | 7.1 mg / 100 kcal |
| Cu | 75 µg / 100 kcal |
| Mn | 65 µg / 100 kcal |
| Mo | 4.0 µg / 100 kcal |
| Se | 3.0 µg / 100 kcal |
| Cr | 2.5 µg / 100 kcal |
| I | 130 µg / 100 kcal |

| **VITAMINS** | |
|---|---|
| Vitamin A | 41 µg RE/ 100 kcal |
| Vitamin D3 | 1.3 µg / 100 kcal |
| Vitamin E | 1.4-1.6 mg α-TE / 100 kcal |
| Vitamin K1 | 4.0 µg / 100 kcal |
| Thiamin (B1) | 0.15 mg / 100 kcal |
| Riboflavin (B2) | 0.16 mg / 100 kcal |
| Niacin (B3) | 1.1 mg NE/ 100 kcal |
| Pantothenic acid (B5) | 0.33 mg / 100 kcal |
| Vitamin B6 | 0.12 mg / 100 kcal |
| Folic acid | 18 µg / 100 kcal |
| Vitamin B12 | 1.1 µg / 100 kcal |
| Biotin | 3.9 µg / 100 kcal |
| Vitamin C | 10 mg / 100 kcal |
| Choline | 104 mg / 100 kcal |
| Taurine | 7.5 mg / 100 kcal |
| L-Carnitine | 1.9 mg / 100 kcal |
| Inositol | 0 mg / 100 kcal |
| Carotenoids | 0.1 mg / 100 kcal |
| UMP | 14.4 mg / 100 kcal |
| CMP | 14.4 mg / 100 kcal |
| Total nucleotides | 28.8 mg / 100 kcal |

Another nutritional composition according to the invention is a formula to be used as supplement (once daily) for infants from birth up to 12 months of age, where children should get 20 ml/kg bodyweight per day. 200ml of formula for a child of 10 kg provide additionally 378mg DHA, 105mg choline and 18mg UMP. An infant should receive 20ml/kg/day. Table 10 shows an exemplary serving for a child of 10 kg.

**Table 10.**

| *Nutrients* | **nutritional value for a once daily formula for a 10 kg child (200 ml serving)** |
|---|---|
| **MACRO NUTRIENTS** | |
| Energy | 150 kcal (75 kcal / 100ml) |
| Protein | 2.68 g / 100 kcal (10.8 en%) |
| Fat | 5.34 g / 100 kcal |
| Linoleic acid | 691 mg / 100 kcal |
| Alpha-linolenic acid | 97.1 mg / 100 kcal |
| AA | 52 mg / 100 kcal |
| EPA | 55 mg / 100 kcal |
| DHA | 270 mg / 100 kcal |
| Carbohydrates | 9.93 g / 100 kcal |
| Lactose | 7.78 g / 100 kcal |
| GOS/long chain FOS | 0.76 g / 100 kcal |

| **MINERALS** | |
|---|---|
| Na | 37.6 mg / 100 kcal |
| K | 104 mg / 100 kcal |
| Cl | 70.1 mg / 100 kcal |
| Ca | 117 mg / 100 kcal |
| P | 63 mg / 100 kcal |
| Mg | 9.37 mg / 100 kcal |

| **TRACE ELEMENTS** | |
|---|---|
| Fe | 1.61 mg / 100 kcal |
| Zn | 6.3 mg / 100 kcal |
| Cu | 80.2 µg / 100 kcal |
| Mn | 13.4 µg / 100 kcal |
| Se | 2.28 µg / 100 kcal |
| I | 127 µg / 100 kcal |

| **VITAMINS** | |
|---|---|
| Vitamin A | 134 µg RE / 100 kcal |
| Vitamin D3 | 2.28 µg / 100 kcal |
| Vitamin E | 2.95 mg α-TE / 100 kcal |
| Vitamin K1 | 7.91 µg / 100 kcal |
| Thiamin (B1) | 0.12 mg / 100 kcal |
| Riboflavin (B2) | 0.2 mg / 100 kcal |
| Niacin (B3) | 1.64 mg NE/ 100 kcal |
| Pantothenic acid (B5) | 0.8 mg / 100 kcal |
| Vitamin B6 | 0.11 mg / 100 kcal |
| Folic acid | 26.8 µg / 100 kcal |
| Vitamin B12 | 1.1 µg / 100 kcal |
| Biotin | 4.02 µg / 100 kcal |
| Vitamin C | 16.1 mg / 100 kcal |
| Choline | 87 mg / 100 kcal |
| Taurine | 6.57 mg / 100 kcal |
| L-Carnitine | 1.21 mg / 100 kcal |
| Inositol | 29.5 mg / 100 kcal |
| UMP | 12.9 mg / 100 kcal |
| CMP | 14.2 mg / 100 kcal |
| Total nucleotides | 28.4 mg / 100 kcal |

Another example of a nutritional composition is a formula for infants from birth up to 12 months of age, as shown in table 11, to be used as complete nutrition.

**Table 11.**

| *Nutrients* | **nutritional value for a formula for use as complete nutrition** |
|---|---|
| **MACRO NUTRIENTS** | |
| Energy | 150 kcal (75 kcal / 100ml) |
| Protein | 2.68 g/100 kcal (10.8 en%) |
| Fat | 5.34 g/100 kcal |
| Linoleic acid | 691 mg/100 kcal |
| Alpha-linolenic acid | 97.1 mg/100 kcal |
| AA | 27.8 mg/100 kcal |
| EPA | 10.8 mg/100 kcal |
| DHA | 51.4 mg/100 kcal |
| Carbohydrates | 9.93 g/100 kcal |
| Lactose | 7.78 g/ 100 kcal |
| GOS/long chain FOS | 0.76 g / 100 kcal |

| **MINERALS** | |
|---|---|
| Na | 37.6 mg / 100 kcal |
| K | 104 mg / 100 kcal |
| Cl | 70.1 mg / 100 kcal |
| Ca | 117 mg / 100 kcal |
| P | 63 mg / 100 kcal |
| Mg | 9.37 mg / 100 kcal |

| **TRACE ELEMENTS** | |
|---|---|
| Fe | 1.61 mg / 100 kcal |
| Zn | 1.88 mg / 100 kcal |
| Cu | 80.2 µg / 100 kcal |
| Mn | 13.4 µg / 100 kcal |
| Se | 2.28 µg / 100 kcal |
| I | 40.1 µg / 100 kcal |

| **VITAMINS** | |
|---|---|
| Vitamin A | 134 µg RE/ 100 kcal |
| Vitamin D3 | 2.28 µg / 100 kcal |
| Vitamin E | 2.95 mg α-TE / 100 kcal |
| Vitamin K1 | 7.91 µg / 100 kcal |
| Thiamin (B1) | 0.12 mg / 100 kcal |
| Riboflavin (B2) | 0.2 mg / 100 kcal |
| Niacin (B3) | 1.64 mg NE / 100 kcal |
| Pantothenic acid (B5) | 0.8 mg / 100 kcal |
| Vitamin B6 | 0.11 mg / 100 kcal |
| Folic acid | 26.8 µg / 100 kcal |
| Vitamin B12 | 0.41 µg / 100 kcal |
| Biotin | 4.02 µg / 100 kcal |
| Vitamin C | 16.1 mg / 100 kcal |
| Choline | 26.7 mg / 100 kcal |
| Taurine | 6.57 mg / 100 kcal |
| L-Carnitine | 1.21 mg / 100 kcal |
| Inositol | 29.5 mg / 100 kcal |
| UMP | 2.47 mg / 100 kcal |
| CMP | 3.76 mg / 100 kcal |
| Total nucleotides | 7.51 mg / 100 kcal |

### Example 4. Compositions / products.

Referring to Table 12, all possible combinations (including combinations of the different ranges) are envisaged and encompassed in the present invention. The ranges are per 100 kcal and/or per 100 ml; more preferably per 100 kcal.

**Table 12. Preferred compositions and combinations, expressed per 100 kcal or per 100 ml product.**

| **Nutrients** | | | **Most preferred range per 100 kcal and/or 100 ml.** |
|---|---|---|---|
| UMP | | | 10 - 200 mg |
| Choline | | | 60 - 350 mg |
| DHA + EPA | | | 250 - 9000 mg |
| Preferably DHA | | | 250 - 900 mg |
| Preferably EPA | | | 50 - 150 mg |
| Optionally vitamin B12 | | | 1 - 5 mcg |
| Optionally iodine | | | 60 - 150 mcg |
| Optionally zinc | | | 5 - 25 mg |

## Claims

1. A composition comprising therapeutically effective amounts of (i) one or more of uridine and cytidine, or salts, phosphates, acyl derivatives or esters thereof, (ii) at least one of docosahexaenoic acid (22:6; DHA), eicosapentaenoic acid (20:5; EPA) and docosapentaenoic acid (22:5; DPA), or esters thereof, (iii) choline, or salts or esters thereof, for use in therapeutically improving language development and reducing language skills impairment in children with suspected or confirmed cerebral palsy, or children at increased risk thereof, wherein said composition comprises:
(i) 10 - 200 mg uridine monophosphate (UMP) per 100 kcal or per 100 ml; and
(ii) 250 - 9000 mg DHA + EPA per 100 kcal or per 100 ml; and
(iii) 60 - 350 mg choline per 100 kcal or per 100 ml.

2. A combination of therapeutically effective amounts of (i) one or more of uridine and cytidine, or salts, phosphates, acyl derivatives or esters thereof, (ii) at least one of docosahexaenoic acid (22:6; DHA), eicosapentaenoic acid (20:5; EPA) and docosapentaenoic acid (22:5; DPA), or esters thereof, (iii) choline, or salts or esters thereof, for use in therapeutically improving language development and reducing language skills impairment in children with suspected or confirmed cerebral palsy, or children at risk thereof, wherein said combination comprises:
(i) 10 - 200 mg UMP per 100 kcal or per 100 ml, and
(ii) 250 - 9000 mg DHA + EPA per 100 kcal or per 100 ml; and
(iii) 60 - 350 mg choline per 100 kcal or per 100 ml..

3. The composition or combination for use according to claim 1 or 2, further comprising therapeutic amounts of vitamin B12, including its functional equivalents.

4. The composition or combination for use according to any of the preceding claims, further comprising therapeutic amounts of iodine.

5. The composition or combination for use according to any of the preceding claims, further comprising therapeutic amounts of zinc.

6. The composition or combination for use according to any of the preceding claims, wherein the child suffers from cerebral palsy and/or has an age between 1 month and 12 years, preferably between 1 and 42 months.

7. The composition or combination for use according to any of the preceding claims, wherein the child at risk of cerebral palsy was born preterm and small for gestational age; or born preterm with brain injury; or born term but small for gestational age; or born term with brain injury, or born preterm and small for gestational age with brain injury or born term but small for gestational age with brain injury.

8. The composition or combination for use according to any of the preceding claims, wherein DHA is administered in a daily amount of 32.5 - 65 mg/kg body weight, preferably 35 - 62.0 mg/kg body weight, more preferably 36-60 mg/kg body weight, even more preferably 36-50 mg/kg bodyweight, most preferably 36-41 mg/kg body weight.

9. The composition or combination for use according to any of the preceding claims, wherein said improvement of language development or said reduction in language skills impairment is measured at the language scale of the Bayley III test.

10. The composition or combination for use according to any of the preceding claims, wherein said child prior to treatment has a Bayley III language composite score of less than 85.

11. The composition or combination for use according to any of the preceding claims, wherein the total daily dosage of DHA+EPA+DPA is in the range of 35 - 75 mg/kg body weight, preferably 40 - 72.5 mg/kg body weight, preferably 43.5 - 72.5 mg/kg body weight, preferably 43.5 - 60 mg/kg body weight, more preferably 43 - 49.5 mg/kg bodyweight.

12. The composition or combination for use according to any of the preceding claims, wherein uridine, as the cumulative amount of uridine, deoxyuridine, uridine phosphates, nucleobase uracil and acylated uridine derivatives, is administered in a daily amount of 1.0 - 4.0 mg per kg body weight, preferably 1.0 - 3.5 mg per kg body weight, more preferably 1.5 - 3.0 mg per kg body weight, more preferably 1.8 - 3.0 mg/kg body weight, even more preferably 1.8 - 2.0 mg/kg body weight.

13. The composition or combination for use according to any of the preceding claims, wherein the total daily dosage of choline, preferably as choline salt, is 7.5 to 35 mg, more preferably 8 to 31 mg, more preferably 9 - 31 mg per kg body weight, most preferably 9 to 14 mg choline per kg body weight.

## Patentansprüche

1. Zusammensetzung umfassend therapeutisch wirksame Mengen an (i) einem oder mehr aus Uridin und Cytidin, oder Salzen, Phosphaten, Acylderivaten oder Estern davon, (ii) zumindest einem aus Docosahexaensäure (22:6; DHA), Eicosapentaensäure (20:5; EPA) und Docosapentaensäure (22:5; DPA), oder Estern davon, (iii) Cholin, oder Salzen oder Estern davon, zur Anwendung in der therapeutischen Verbesserung der Sprachentwicklung und Verringerung der Sprachfähigkeitsbeeinträchtigung bei Kindern mit Verdacht auf oder bestätigter Zerebralparese, oder Kindern mit erhöhtem Risiko dafür, wobei besagte Zusammensetzung umfasst:
(i) 10 - 200 mg Uridinmonophosphat (UMP) pro 100 kcal oder pro 100 mL; und
(ii) 250 - 9000 mg DHA + EPA pro 100 kcal oder pro 100 mL; und
(iii) 60 - 350 mg Cholin pro 100 kcal oder pro 100 mL.

2. Kombination therapeutisch wirksamer Mengen an (i) einem oder mehr aus Uridin und Cytidin, oder Salzen, Phosphaten, Acylderivaten oder Estern davon, (ii) zumindest einem aus Docosahexaensäure (22:6; DHA), Eicosapentaensäure (20:5; EPA) und Docosapentaensäure (22:5; DPA), oder Estern davon, (iii) Cholin, oder Salzen oder Estern davon, zur Anwendung in der therapeutischen Verbesserung der Sprachentwicklung und Verringerung der Sprachfähigkeitsbeeinträchtigung bei Kindern mit Verdacht auf oder bestätigter Zerebralparese, oder Kindern mit erhöhtem Risiko dafür, wobei besagte Kombination umfasst:
(i) 10 - 200 mg Uridinmonophosphat (UMP) pro 100 kcal oder pro 100 mL, und
(ii) 250 - 9000 mg DHA + EPA pro 100 kcal oder pro 100 mL; und
(iii) 60 - 350 mg Cholin pro 100 kcal oder pro 100 mL.

3. Zusammensetzung oder Kombination zur Anwendung gemäß Anspruch 1 oder 2, ferner umfassend therapeutische Mengen an Vitamin B12, einschließlich seiner funktionalen Äquivalente.

4. Zusammensetzung oder Kombination zur Anwendung gemäß einem der vorhergehenden Ansprüche, ferner umfassend therapeutische Mengen an Iod.

5. Zusammensetzung oder Kombination zur Anwendung gemäß einem der vorhergehenden Ansprüche, ferner umfassend therapeutische Mengen an Zink.

6. Zusammensetzung oder Kombination zur Anwendung gemäß einem der vorhergehenden Ansprüche, wobei das Kind unter Zerebralparese leidet und/oder zwischen 1 Monat und 12 Jahren alt ist, bevorzugt zwischen 1 und 42 Monaten.

7. Zusammensetzung oder Kombination zur Anwendung gemäß einem der vorhergehenden Ansprüche, wobei das Kind mit Risiko für Zerebralparese als Frühgeburt und klein für das Schwangerschaftsalter geboren wurde; oder als Frühgeburt mit einer Hirnschädigung geboren wurde; oder termingeboren wurde aber klein für das Schwangerschaftsalter; oder termingeboren wurde mit einer Hirnschädigung, oder als Frühgeburt und klein für das Schwangerschaftsalter mit einer Hirnschädigung geboren wurde oder termingeboren wurde aber klein für das Schwangerschaftsalter mit einer Hirnschädigung.

8. Zusammensetzung oder Kombination zur Anwendung gemäß einem der vorhergehenden Ansprüche, wobei DHA in einer täglichen Menge von 32,5 - 65 mg/kg Körpergewicht, bevorzugt 35 - 62,0 mg/kg Körpergewicht, mehr bevorzugt 36 - 60 mg/kg Körpergewicht, noch mehr bevorzugt 36 - 50 mg/kg Körpergewicht, am meisten bevorzugt 36 - 41 mg/kg Körpergewicht verabreicht wird.

9. Zusammensetzung oder Kombination zur Anwendung gemäß einem der vorhergehenden Ansprüche, wobei besagte Verbesserung der Sprachentwicklung oder besagte Verringerung der Sprachfähigkeitsbeeinträchtigung an der Sprachskala des Bayley III-Tests gemessen wird.

10. Zusammensetzung oder Kombination zur Anwendung gemäß einem der vorhergehenden Ansprüche, wobei besagtes Kind vor der Behandlung ein zusammengesetztes Bayley III-Sprachergebnis von weniger als 85 aufweist.

11. Zusammensetzung oder Kombination zur Anwendung gemäß einem der vorhergehenden Ansprüche, wobei die tägliche Gesamtdosierung an DHA+EPA+DPA im Bereich von 35 - 75 mg/kg Körpergewicht, bevorzugt 40 bis 72,5 mg/kg Körpergewicht, bevorzugt 43,5 - 72,5 mg/kg Körpergewicht, bevorzugt 43,5 - 60 mg/kg Körpergewicht, mehr bevorzugt 43 - 49,5 mg Körpergewicht ist.

12. Zusammensetzung oder Kombination zur Anwendung gemäß einem der vorhergehenden Ansprüche, wobei Uridin, als die kumulative Menge an Uridin, Desoxyuridin, Uridinphosphaten, nukleobasischem Uracil und acylierten Uridinderivaten, in einer täglichen Menge von 1,0 - 4,0 mg pro kg Körpergewicht, bevorzugt 1,0 - 3,5 mg pro kg Körpergewicht, mehr bevorzugt 1,5 - 3,0 mg pro kg Körpergewicht, mehr bevorzugt 1,8 - 3,0 mg/kg Körpergewicht, noch mehr bevorzugt 1,8 - 2,0 mg/kg Körpergewicht verabreicht wird.

13. Zusammensetzung oder Kombination zur Anwendung gemäß einem der vorhergehenden Ansprüche, wobei die tägliche Gesamtdosierung an Cholin, bevorzugt als Cholinsalz, 7,5 bis 35 mg, mehr bevorzugt 8 bis 31 mg, mehr bevorzugt 9 bis 31 mg pro kg Körpergewicht, am meisten bevorzugt 9 - 14 mg Cholin pro kg Körpergewicht ist.

## Revendications

1. Composition comprenant des quantités thérapeutiquement efficaces (i) d'un ou de plusieurs composés parmi l'uridine et la cytidine, ou de sels, phosphates, dérivés acylés ou esters de celles-ci, (ii) au moins un composé parmi l'acide docosahexaénoïque (22:6 ; DHA), l'acide eicosapentaénoïque (20:5 ; EPA) et l'acide docosapentaénoïque (22:5 ; DPA) ou des esters de ceux-ci ; (iii) de choline, ou de sels ou esters de celle-ci, pour une utilisation dans l'amélioration thérapeutique du développement du langage et la réduction de la dégradation des capacités langagières chez les enfants présentant une paralysie cérébrale suspectée ou confirmée, ou chez les enfants présentant un risque accru de paralysie cérébrale, ladite composition comprenant :
(i) 10 à 200 mg de monophosphate d'uridine (UMP) par 100 kcal ou par 100 ml ; et
(ii) 250 à 9 000 mg de DHA + EPA par 100 kcal ou par 100 ml ; et
(iii) 60 à 350 mg de choline par 100 kcal ou par 100 ml.

2. Combinaison de quantités thérapeutiquement efficaces (i) d'un ou de plusieurs composés parmi l'uridine et la cytidine, ou les sels, phosphates, dérivés acylés ou esters de celles-ci, (ii) d'au moins un composé parmi l'acide docosahexaénoïque (22:6 ; DHA), l'acide eicosapentaénoïque (20:5 ; EPA) et l'acide docosapentaénoïque (22:5 ; DPA) ou des esters de ceux-ci, (iii) de choline, ou de sels ou esters de celle-ci, pour une utilisation dans l'amélioration thérapeutique du développement du langage et la réduction de la dégradation des capacités langagières chez les enfants présentant une paralysie cérébrale suspectée ou confirmée ou chez les enfants présentant un risque accru de paralysie cérébrale, ladite combinaison comprenant :
(i) 10 à 200 mg de monophosphate d'uridine (UMP) par 100 kcal ou par 100 ml ; et
(ii) 250 à 9 000 mg de DHA + EPA par 100 kcal ou par 100 ml ; et
(iii) 60 à 350 mg de choline par 100 kcal ou par 100 ml.

3. Composition ou combinaison pour une utilisation selon la revendication 1 ou 2, comprenant en outre des quantités thérapeutiques de vitamine B12, y compris ses équivalents fonctionnels.

4. Composition ou combinaison pour une utilisation selon l'une quelconque des revendications précédentes, comprenant en outre des quantités thérapeutiques d'iode.

5. Composition ou combinaison pour une utilisation selon l'une quelconque des revendications précédentes, comprenant en outre des quantités thérapeutiques de zinc.

6. Composition ou combinaison pour une utilisation selon l'une quelconque des revendications précédentes, l'enfant souffrant de paralysie cérébrale et/ou ayant un âge compris entre 1 mois et 12 ans, de préférence entre 1 et 42 mois.

7. Composition ou combinaison pour une utilisation selon l'une quelconque des revendications précédentes, l'enfant présentant un risque de paralysie cérébrale étant né avant terme et étant petit pour son âge gestationnel ; ou né avant terme avec lésion cérébrale ; ou né à terme mais petit pour son âge gestationnel ; ou né à terme avec une lésion cérébrale, ou né avant terme et petit pour son âge gestationnel avec lésion cérébrale ou né à terme mais petit pour son âge gestationnel et avec lésion cérébrale.

8. Composition ou combinaison pour une utilisation selon l'une quelconque des revendications précédentes, le DHA étant administré en une quantité journalière de 32,5 à 65 mg/kg de poids corporel, de préférence de 35 à 62,0 mg/kg de poids corporel, plus préférentiellement de 36 à 60 mg/kg de poids corporel, d'une manière encore plus préférée de 36 à 50 mg/kg de poids corporel, tout spécialement de 36 à 41 mg/kg de poids corporel.

9. Composition ou combinaison pour une utilisation selon l'une quelconque des revendications précédentes, dans laquelle ladite amélioration du développement du langage ou ladite réduction de la dégradation des capacités langagières est mesurée sur l'échelle de langage du test Bayley III.

10. Composition ou combinaison pour une utilisation selon l'une quelconque des revendications précédentes, ledit enfant avant le traitement ayant un score composite de langage Bayley III inférieur à 85.

11. Composition ou combinaison pour une utilisation selon l'une quelconque des revendications précédentes, dans laquelle la posologie journalière totale de DHA + EPA + DPA est comprise dans la plage de 35 à 75 mg/kg de poids corporel, de préférence de 40 à 72,5 mg/kg de poids corporel, de préférence de 43,5 à 72,5 mg/kg de poids corporel, de préférence de 43,5 à 60 mg/kg de poids corporel, plus préférentiellement de 43 à 49,5 mg/kg de poids corporel.

12. Composition ou combinaison pour une utilisation selon l'une quelconque des revendications précédentes, dans laquelle l'uridine, exprimée par la quantité cumulée d'uridine, de désoxyuridine, de phosphates d'uridine, de la nucléobase uracil et des dérivés acylés de l'uridine, est administrée en une quantité journalière de 1,0 à 4,0 mg par kg de poids corporel, de préférence de 1,0 à 3,5 mg par kg de poids corporel, plus particulièrement de 1,5 à 3,0 mg par kg de poids corporel, plus préférentiellement de 1,8 à 3,0 mg/kg de poids corporel, d'une manière encore plus préférée de 1,8 à 2,0 mg/kg de poids corporel.

13. Composition ou combinaison pour une utilisation selon l'une quelconque des revendications précédentes, la posologie journalière totale de choline, de préférence exprimée en sel de choline, étant de 7,5 à 35 mg, plus préférentiellement de 8 à 31 mg, plus préférentiellement de 9 à 31 mg par kg de poids corporel, tout spécialement de 9 à 14 mg de choline par kg de poids corporel.
